# EUROPEAN PATENT APPLICATION

(11) **EP 3 895 534 A1**
(43) Date of publication of application: **20.10.2021**
(21) Application number: 21168332.1
(22) Date of filing: 14.04.2021
(51) Int. Cl.: A01N 1/02, A61B 50/00, A61F 7/10

(54) **ORGAN CONTAINER**

(30) Priority: 14.04.2020 JP 2020072203
(71) Applicant: SCREEN Holdings Co., Ltd., Kyoto 602-8585 (JP)
(72) Inventor: TORAI, Shinji, KYOTO-SHI, Kyoto 602-8585 (JP); YOSHIMOTO, Syuhei, KYOTO-SHI, Kyoto 602-8585 (JP)
(74) Representative: Kilian Kilian & Partner

(57) **Abstract**

An organ container (1) for accommodating an organ, comprising:

a contractible and expandable pouch-shaped body (20) having an opening (21), wherein, under a no-load condition, said opening (21) has a maximum width smaller than a maximum width of said body (20),

said opening (21) is expandable up to a state in which the maximum width of said opening (21) is greater than the maximum width of said body, and

said body (20) has an inner surface (200) including:

a first region (30) having a smooth curved surface; and

a second region (40) having an uneven shape.

## Description

### BACKGROUND OF THE INVENTION

### Field of the invention

The present invention relates to an organ container for accommodating an organ.

### Description of the Background Art

In organ transplant operations, an organ removed from a donor is preserved under cooled conditions. This is because if the organ is left at ordinary temperature and the blood flowing from and to the organ is stopped, i.e., the organ gets into a so-called warm ischemic state, the organ becomes easy to deteriorate due to metabolism in the organ. Specifically, the temperature of the organ is kept low through procedures such as pouring a low-temperature preservation solution into the isolated organ or directly spraying ice-slush saline around the organ. This suppresses organ metabolism.

However, at the time of transplanting an organ into a recipient, the organ is placed in the body cavity of the recipient and undergoes procedures such as vascular anastomosis. At this time, the organ cannot be kept under cooled conditions, so that the temperature of the organ rises due to the body temperature of the recipient or the outside air temperature and the organ gradually gets into a warm ischemic state. Hence, a surgeon carrying out the transplant operation has to perform procedures such as vascular anastomosis within a period of time as short as possible or to maintain low-temperature conditions of the organ to be transplanted by, for example, pouring ice or other materials into the abdominal cavity. In the latter case, not only the organ but also the fingertips of the surgeon will be cooled at the same time, and this is disadvantageous for vascular anastomosis that requires high precision.

In view of this, the inventors of the present application have proposed a technique disclosed in Japanese Patent Application Laid-Open No. 2018-000309 in which at the time of transplanting an organ into a recipient, a sheet having an insulating function is inserted between the recipient and the organ to suppress a temperature rise in the organ. The sheet according to Japanese Patent Application Laid-Open No. 2018-000309 includes an adiabatic layer and two water-repellent layers that are face-adhered to the opposite sides of the adiabatic layer.

However, the sheet according to Japanese Patent Application Laid-Open No. 2018-000309 does not completely cover the organ. Thus, there is a problem that a temperature rise occurs in the portion of the surface of the organ that is not covered with the sheet, resulting in organ deterioration.

### SUMMARY OF THE INVENTION

It is an object of the present invention to provide a technique for more reliably suppressing a temperature rise in an organ and reducing damage to the organ during transplantation of the organ to a recipient.

In order to solve the problem described above, a first aspect of the present application is an organ container for accommodating an organ, and includes a contractible and expandable pouch-shaped body having an opening. Under a no-load condition, the opening has a maximum width smaller than a maximum width of the body. The opening is expandable up to a state in which the maximum width of the opening is greater than the maximum width of the body. The body has an inner surface including a first region having a smooth curved surface, and a second region having an uneven shape.

According to the first aspect of the present application, the uneven shape of the inner surface of the body allows a preservation solution to penetrate into interstices between the organ and the inner surface and thereby improves a thermal insulating effect. Moreover, by providing the smooth first region with no projections and depressions in a region of the inner surface of the body where traces are likely to remain, generation of remaining traces can be reduced. Accordingly, it is possible to effectively suppress a temperature rise in the organ and to reduce damage to the organ.

These and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a perspective view of an organ container;
Fig. 2 is a top view of the organ container;
Fig. 3 is a longitudinal sectional view of the organ container;
Fig. 4 is a cross-sectional view of the organ container;
Fig. 5 is a diagram illustrating how an organ is accommodated in the organ container;
Fig. 6 is a diagram illustrating how the organ is accommodated in the organ container;
Fig. 7 is a diagram illustrating how the organ is accommodated in the organ container;
Fig. 8 is a flowchart of a procedure for a transplant operation using the organ container;
Fig. 9 is a longitudinal sectional view of an organ container according to one variation;
Fig. 10 is a cross-sectional view of an organ container according to the variation;.
Fig. 11 is a longitudinal sectional view of an organ container according to another variation;
Fig. 12 is a longitudinal sectional view of an organ container according to another variation;
Fig. 13 is a longitudinal sectional view of an organ container according to another variation;
Fig. 14A is a partial cross-sectional view of an organ container according to another variation;
Fig. 14B is a partial cross-sectional view of an organ container according to another variation; and
Fig. 14C is a partial cross-sectional view of an organ container according to another variation.

### DESCRIPTION OF THE PREFERRED EMBODIMENT

An embodiment of the present invention will be described hereinafter with reference to the drawings.

In the present application, "donors" and "recipients" may be humans, or may be non-human animals. That is, "organs" according to the present application may be human organs, or may be organs of non-human animals. The non-human animals may be rodents such as mice and rats, ungulates such as pigs, goats, and sheep, non-human primates such as chimpanzees, or other non-human mammals, or may also be nonmammalian animals.

### 1. First Embodiment

### 1-1. Organ Container

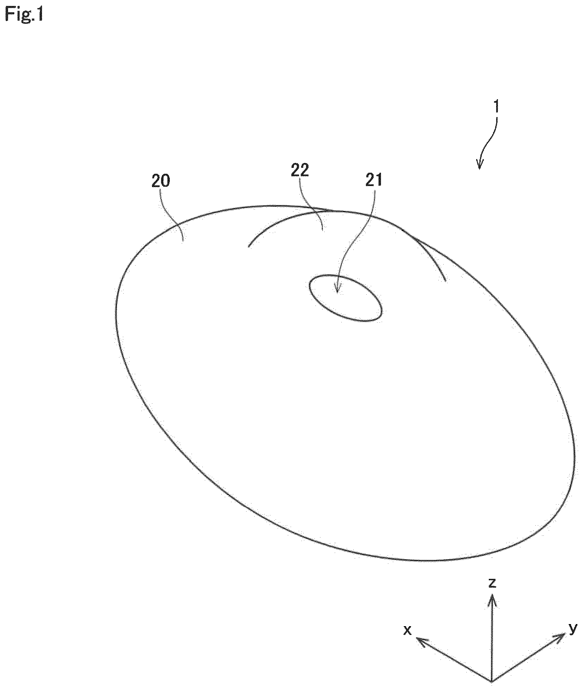

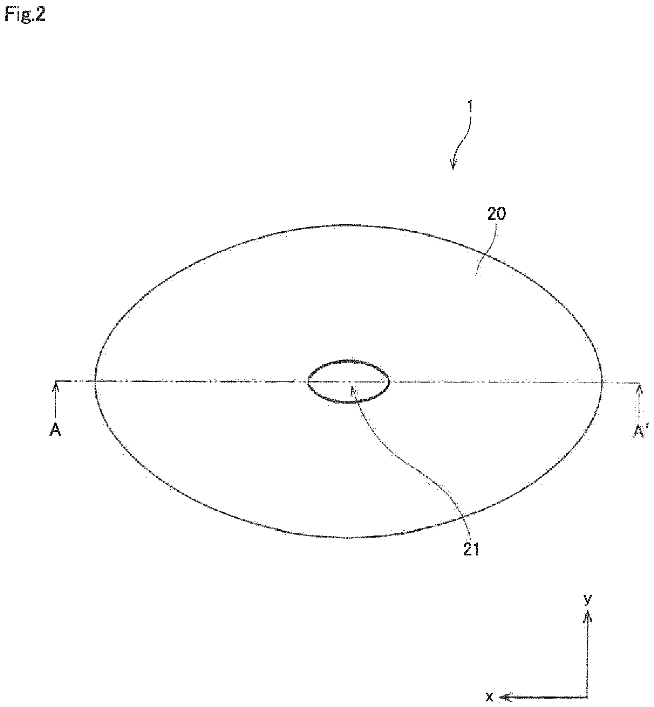

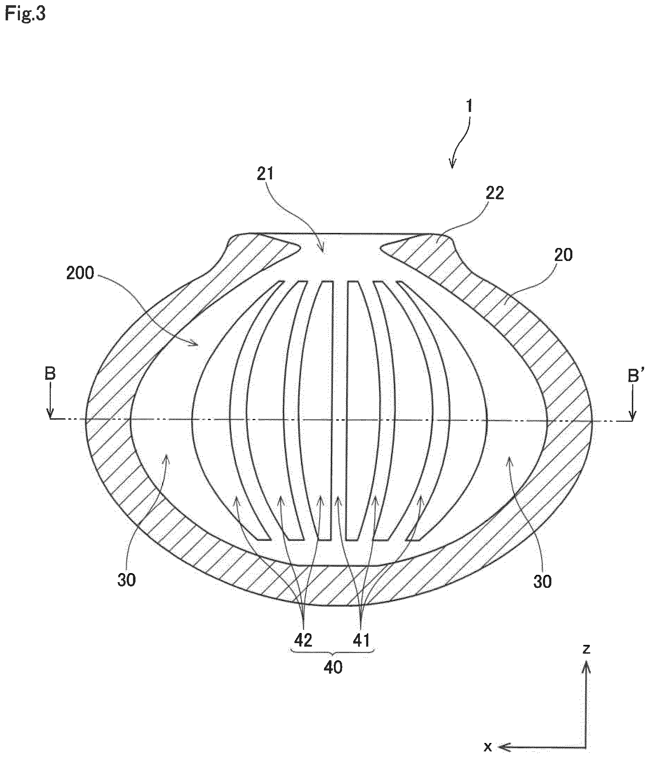

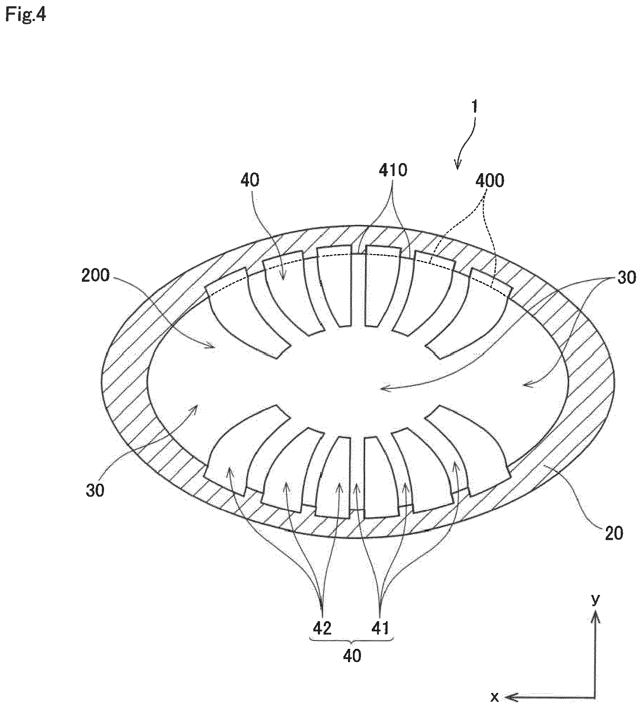

Fig. 1 is a perspective view of an organ container 1 according to a first embodiment. Fig. 2 is a top view of the organ container 1. Fig. 3 is a longitudinal sectional view of the organ container 1. Fig. 4 is a cross-sectional view of the organ container 1. Specifically, Fig. 3 is a sectional view taken along arrows A-A'. Fig. 4 is a sectional view taking along arrows B-B'. This organ container 1 is a container for temporarily accommodating an organ removed from a donor in a transplant operation for transplanting the organ into the recipient. That is, the organ container 1 is a medical appliance for use in organ transplant operations.

Examples of organs that can be accommodated in the organ container 1 include a kidney, a heart, and a lung. Blood vessels of these organs that are to be anastomosed in transplant operations are concentrated on one side of the organs. The structure of the organ container 1 according to the present embodiment is particularly suitable for such organs. However, the organ container according to the present invention may be configured to accommodate any other organ such as a liver.

In the following description, an up-down direction is defined such that the side of the organ container 1 with an opening 21 is regarded as the upper side and the bottom side of the organ container 1 opposite to the opening 21 is regarded as the lower side. In the following description, as illustrated in Fig. 1, the up-down direction is also referred to as a z direction, a direction along the long sides of the organ container 1 looking in the z direction is referred to as an x direction, and a direction along the short sides of the organ container 1 looking in the z direction is referred to as a y direction. Note that the definition of the up-down direction does not intend to limit the orientation of the organ container 1 during use.

As illustrated in Fig. 1, the organ container 1 includes a contractible and expandable pouch-shaped body 20. The body 20 has an opening 21 and a thick portion 22. The opening 21 communicates with the internal space of the body 20 and an exterior space. The thick portion 22 surrounds the opening 21 like a ring.

The organ container 1 according to the present embodiment has a generally ellipsoidal shape as a whole. That is, the body 20 has a generally ellipsoidal shape. This organ container 1 is particularly configured to accommodate a kidney. The organ container 1 as a whole is made in a generally ellipsoidal shape so that the inner surface of the body 20 has a shape that can easily fit along the surface of a kidney.

The body 20 is formed of an elastomer gel. Specifically, the body 20 is formed of a thermoplastic elastomer, an urethane elastomer, or an oil bleeding silicone gel. The body 20 has a hardness of, for example, E10 to A10 according to a durometer hardness test compliant with Japanese Industrial Standards JIS K 6253-3:2012. The body 20 has an elongation percentage higher than or equal to 1000% at breakage.

By forming the body 20 of such a material, the body 20 and the opening 21 are easy to expand and less likely to break. By forming the body 20 of such a material, it is possible to reduce the possibility that the temperature outside the body 20 propagates to the organ in the body 20 when the organ is accommodated in the body 20.

The opening 21 is provided for inserting an organ into the internal space of the body 20. The opening 21 also plays a role as a connection port for connecting blood vessels or other pathways connected to the organ to the outside while the organ is accommodated in the body 20. In the present embodiment, the opening 21 has an elliptical shape. As illustrated in Fig. 2, the major axis of the opening 21 looking in the z direction is along the x direction, like the major axis of the body 20.

The thick portion 22 is a partial portion having a greater thickness than surrounding portions. With increasing thickness, the elastomer gel forming the body 20 has a higher capability to contract in a direction returning to its original shape against loads applied in the direction of expansion. Thus, by surrounding the opening 21 like a ring by the thick portion 22, it is possible to increase the amount of elastic deformation of portions around the opening 21 and to improve strength. That is, when the opening 21 is expanded, it is possible to suppress plastic deformation or breakage of portions around the opening 21.

Besides, the thick portion 22 comes in intimate contact with the organ when the organ is accommodated in the container. This suppresses a protrusion of the organ from the opening 21 and suppresses an outflow of a liquid stored between the organ and the body 20 from the opening 21.

The body 20 has an inner surface 200 that includes first regions 30 having smooth curved surfaces and second regions 40 having uneven shapes. As illustrated in Figs. 3 and 4, in the present embodiment, the first regions 30 are arranged in the bottom of the inner surface 200 that faces the opening 21 and in both end portions of the inner surface 200 in the x axial direction. The second regions 40 are arranged in the other portions.

The second regions 40 includes projections 41 and depressions 42. The projections 41 project toward the internal space of the body 20 inward of adjacent depressions 42. The depressions 42 are depressed toward the outside of the body 20 outward of adjacent projections 41. In the present embodiment, the depressions 42 are configured by a plurality of grooves extending in the z direction along the inner surface 200.

In the second regions 40, a virtual plane on the curved surface contiguous to the adjacent first regions 30 is referred to as a reference plane 400. In Fig. 4, the reference plane 400 is indicated by a broken line. In the present embodiment, inwardly facing surfaces 410 of the projections 41 are on the reference plane 400. Then, the depressions 42 are depressed toward the outside from the reference plane 400.

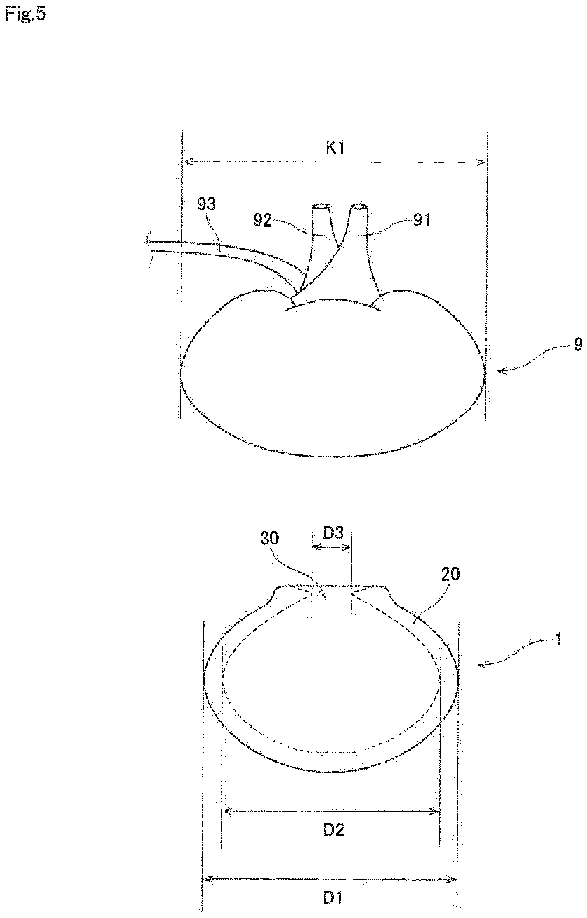

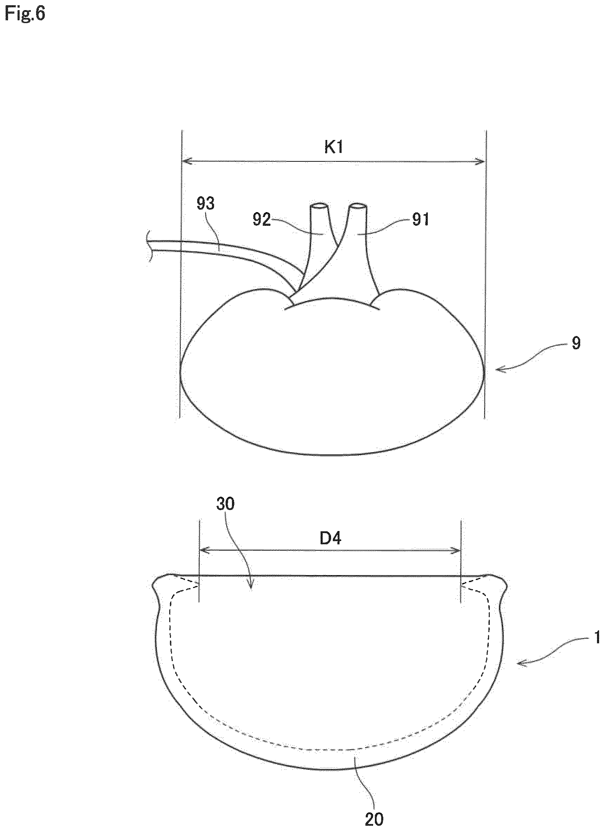

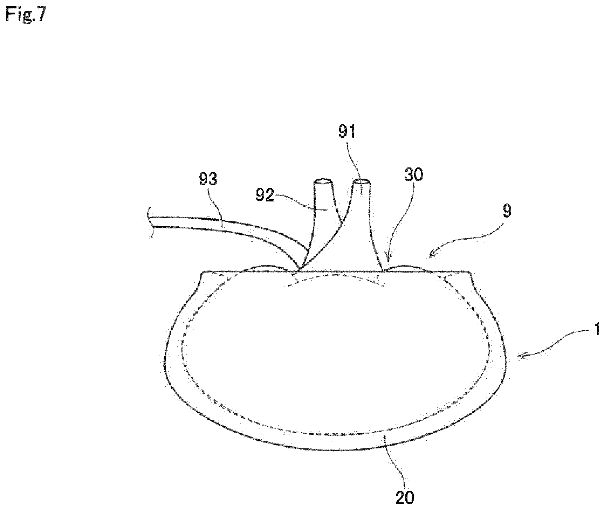

Figs. 5 to 7 are diagrams illustrating how a kidney 9, which is one example of the organ, is accommodated in the organ container 1. Specifically, Fig. 5 is a diagram illustrating the kidney 9 and the organ container 1 under no-load conditions, placed above and below. Fig. 6 is a diagram illustrating the opening 21 of the organ container 1 that is expanded in order to accommodate the kidney 9. Fig. 7 is a diagram illustrating the kidney 9 accommodated in the organ container 1.

As illustrated in Fig. 5, under no-load conditions, the body 20 of the organ container 1 has a maximum width, i.e., a length D1 in the axis direction, smaller than a maximum width, i.e., a length K1 of the kidney 9. That is, under no-load conditions, the inner surface 200 of the body 20 has a maximum width, i.e., length D2, smaller than the maximum width, i.e., length K1, of the kidney 9. Accordingly, under no-load conditions, the opening 21 has a maximum width, i.e., length D3, smaller than the maximum width, i.e., length K1, of the kidney 9.

The opening 21 is expandable up to a state in which the maximum width of the opening 21 is greater than the maximum width, i.e., length D1, of the body 20 under no-load conditions. Thus, the kidney 9 can be easily accommodated in the body 20. Fig. 6 shows that the opening 21 is expanded to a state in which the maximum width of the opening 21 becomes a length D4 greater than the length D1. In this way, an operator expands the opening 21 to accommodate the kidney 9 in the body 20 through the opening 21.

In the organ container 1 according to the present embodiment, the opening 21 is expandable up to a state in which the maximum width of the opening 21 is greater than the maximum width, i.e., length K1, of the kidney 9. Thus, the kidney 9 can be more easily accommodated in the body 20. However, even in the case where the opening 21 cannot be expanded to a state in which the maximum width of the opening 21 is greater than the length K1, it is sufficient that the body 20 can be expanded up to a state in which the maximum width of the inner surface 200 is greater than the length K1.

Then, when the kidney 9 has been accommodated in the body 20 as illustrated in Fig. 7, the entire inner surface 200 of the body 20 including the periphery of the opening 21 fits along the surface of the kidney 9. As described above, under no-load conditions, the inner surface of the body 20 is smaller than the outer surface of the kidney 9. Thus, when the kidney 9 is accommodated in the organ container 1, the inner surface of the body 20 fits along the outer surface of the kidney 9, and the projections 41 on the inner surface of the body 20 come in intimate contact with the outer surface of the kidney 9. Accordingly, the kidney 9 is appropriately held without moving inside the body 20. As a result, damage to the kidney 9 can be reduced. Moreover, because most part of the outer surface of the kidney 9 is covered with the organ container 1 having a thermal insulating effect, it is possible to maintain the temperature of the kidney 9 with ease and to efficiently suppress a temperature rise in the kidney 9.

At the time, the inner surface 200 of the body 20 includes the second regions 40 having uneven shapes as described above. Therefore, a preservation solution for cooling can be held in interstices between the organ and the depressions 42 when the kidney 9 is accommodated in the body 20. In particular, in the case where the depressions 42 include a plurality of grooves extending in the up-down direction as in the present embodiment, the preservation solution for cooling poured from the opening 21 can easily reach the bottom side along the grooves. That is, the preservation solution for cooling held between the organ and the inner surface 200 can easily spread to the entire inner surface. Accordingly, it is possible to efficiently suppress a temperature rise in the kidney 9.

If most part of the inner surface 200 of the body 20 is formed of the second regions 40 having uneven shapes, it is possible to increase the volume of the preservation solution that can be held between the inner surface 200 and the kidney 9. However, since the contractile force of the body 20 causes the inner surface 200 to come into intimate contact with the outer surface of the kidney 9, some portions of the inner surface 200 may be heavily pressed against the kidney 9 depending on the balance of shapes between the kidney 9 and the body 20. If such portions of the inner surface 200 have uneven shapes, traces of the uneven shapes may remain on the outer surface of the kidney 9.

In view of this, in the organ container 1, the smooth first regions 30 with no projections and depressions are arranged in regions where traces are likely to remain. In the case where the kidney 9 is accommodated in the body 20 having a generally ellipsoidal shape such as in the organ container 1, the pressure of the inner surface 200 against the kidney 9 is more likely to be applied in the major axis direction of the ellipsoidal shape, i.e., the x axial direction. Therefore, in the present embodiment, the first regions 30 are arranged in the end portions of the inner surface 200 in the x axial direction. In this way, generation of remaining traces can be suppressed by providing the smooth first regions 30 with no projections and depressions in the regions of the inner surface 200 where traces are likely to remain.

In the present embodiment, the first region 30 with no projections and depressions is also arranged in the vicinity of the bottom of the inner surface 200. This is because there is no particular need to provide an uneven shape in the vicinity of the bottom because the preservation solution for cooling is naturally accumulated in the bottom by gravity.

If the projections 41 of the second regions 40 project inward of the adjacent first regions 30 toward the internal space where the kidney 9 is arranged, traces are likely to remain on the kidney 9. In the present embodiment, as described above, surfaces 410 of the projections 41 on the second regions 40 are on the curved surface (reference plane 400) contiguous to the adjacent first regions 30 as described above. Then, the depressions 42 are depressed toward the outside from the reference plane 400. Accordingly, it is possible to avoid the projections 41 from being engaged in the kidney 9 and thereby to more reliably suppress generation of remaining traces.

### 1-2. Procedure for Organ Transplantation

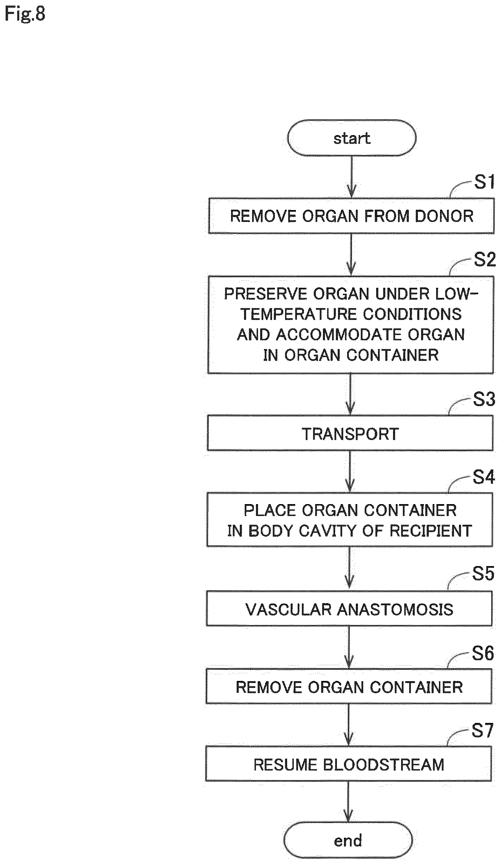

Next, a procedure for a transplant operation using the organ container 1 described above will be described. Fig. 8 is a flowchart illustrating the procedure for a transplant operation using the organ container 1. The following description is given of the case where the kidney 9 is to be transplanted using the organ container 1.

In the transplant operation of a kidney, first, the kidney 9 is removed from a donor (step S1). Specifically, blood vessels 91 and 92 and an ureter 93 that extend from the kidney 9 of the donor (see Figs. 5 to 7) are cut, and the kidney 9 is taken out of the body cavity of the donor.

The removed kidney 9 is preserved while being immersed in a low-temperature preservation solution. The kidney 9 is also accommodated in the organ container 1 (step S2). The preservation solution may, for example, be saline kept at 4°C. If the organ is left at ordinary temperature and the blood flowing from and to the organ is stopped, i.e., the organ gets into a so-called warm ischemia state, the organ becomes easy to deteriorate due to metabolism in the organ. Thus, in step S2, the kidney 9 is preserved at a temperature lower than ordinary temperature in order to suppress deterioration of the kidney 9.

Alternatively, in step S2, tubes may be connected to the blood vessels 91 and 92 of the kidney 9, and the kidney 9 may be preserved while being perfused with a preservation solution. Note that the perfusion using the preservation solution may continue until step S4 described later.

The timing when the kidney 9 is accommodated in the organ container 1 may be before the kidney 9 is immersed in the preservation solution, or may be after the kidney 9 is immersed in the preservation solution for a while and sufficiently cooled. When the kidney 9 is accommodated in the organ container 1, the opening 21 of the organ container 1 is opened, and the kidney 9 is inserted through the opening 21 into the body 20 as illustrated in Fig. 6. Accordingly, the kidney 9 is held inside the pouch-shaped body 20 as illustrated in Fig. 7. At this time, a low-temperature preservation solution is poured into the space between the kidney 9 and the inner surface of the body 20, using a syringe or a pipette. The kidney 9 embraced by the organ container 1 is immersed again in a low-temperature preservation solution to maintain a low-temperature preservation condition.

The kidney 9 held by the organ container 1 is transported from the donor side to the recipient side while being immersed in the low-temperature preservation solution (step S3). The kidney 9 transported to the recipient side continues to be held by the organ container 1 and to be immersed in the low-temperature preservation solution until just before transplantation.

Then, the abdomen of the recipient is opened, and the organ container 1 accommodating therein the kidney 9 is placed in the body cavity of the recipient (step S4). Then, the blood vessels of the recipient are anastomosed to the blood vessels 91 and 92 of the kidney 9 exposed to the outside through the opening 21 of the organ container 1 (step S5). In the case where the organ to be transplanted is the kidney 9, the ureter 93 is also connected to the urinary bladder.

During the work in steps S4 and S5, the kidney 9 accommodated in the organ container 1 is placed within the body cavity. At this time, since the organ container 1 has a thermal insulating function, it is possible to suppress a temperature rise in the kidney 9 due to the body temperature of the recipient or the outside air temperature. This reduces the possibility that the kidney 9 gets into a warm ischemic state and progressively deteriorates due to metabolism. As a result, it is possible to suppress the occurrence of troubles after operation.

In steps S4 and S5, a low-temperature preservation solution is poured at regular time intervals (e.g., every few minutes) into the organ container 1, using a syringe or a pipette. This further suppresses a temperature rise in the kidney 9.

Thereafter, the opening 21 of the organ container 1 is opened, and the kidney 9 that has undergone vascular anastomosis is taken out of the organ container 1. Then, the organ container 1 is removed from the body cavity of the recipient (step S6). Thereafter, the bloodstream from the anastomosed blood vessels of the recipient to the kidney 9 or vice versa is resumed (step S7).

This organ container 1 covers the surface of the kidney 9 along the surface of the kidney 9. Thus, part of the organ container 1 (e.g., end portions) does not expand around the kidney 9. Accordingly, the organ container 1 is less likely to hinder work during operation. Moreover, the organ container 1 covers most part of the surface of the kidney 9, thereby further suppressing a temperature rise in the kidney 9 and preventing damage to the surface of the kidney 9 during operation.

This organ container 1 is formed of a raw material having elasticity. Thus, the organ container 1 can absorb impacts on the kidney 9 during transport or operation. Accordingly, it is possible to reduce damage to the kidney 9.

### 2. Variations

While a principal embodiment of the present invention has been described thus far, the present invention is not intended to be limited to the embodiment described above.

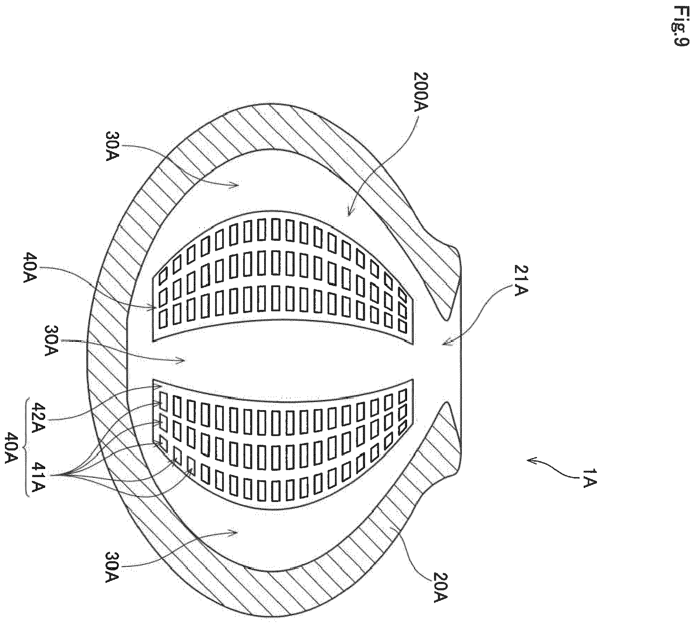

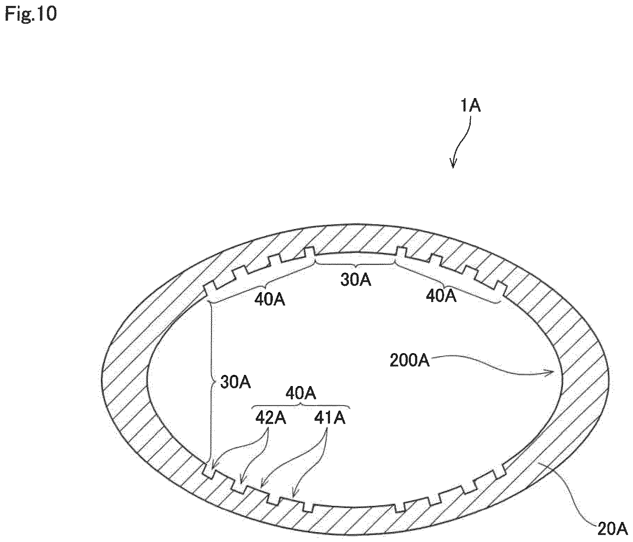

Fig. 9 is a longitudinal sectional view of an organ container 1A according to a variation. Fig. 10 is a cross-sectional view of the organ container 1A. In Fig. 10, only a cross-section of the organ container 1A is illustrated, and an uneven shape of the inner surface 200A on the bottom side of the organ container 1A is not illustrated.

The organ container 1A has a body 20A that has a shape similar to the shape of the body 20 of the organ container 1 according to the first embodiment, except for the uneven shape of an inner surface 200A. Thus, the body 20A of the organ container 1A has a generally ellipsoidal shape and has a generally elliptical cross-section looking from the side of an opening 21A.

As illustrated in Fig. 10, the organ container 1A includes first regions 30A and second regions 40A that are arranged alternately in a cross-section looking from the side of the opening 21A. In this way, by arranging a plurality of second regions 40A having uneven shapes at different locations, it is possible to hold a preservation solution for cooling at different locations in the circumferential direction. This improves the efficiency of thermal insulation of the organ.

In a cross-section of the organ container 1A looking from the side of the opening 21A, the first regions 30A are arranged in both end portions of the inner surface 200A in the major axis direction and both end portions of the inner surface 200A in the minor axis direction. In this way, by arranging the first regions 30A with no projections and depressions in regions where the pressure of the inner surface 200A against the accommodated organ tends to increase, generation of remaining traces can be suppressed efficiently.

In the organ container 1A, the second regions 40A include a plurality of projections 41A arranged at intervals and depressions 42A arranged in the other portions. Thus, the depressions 42A are arranged in a mesh-like fashion in the up-down direction and in the horizontal direction. This allows the preservation solution for cooling when poured from the opening 21A to efficiently spread to the entire of the second regions 40A.

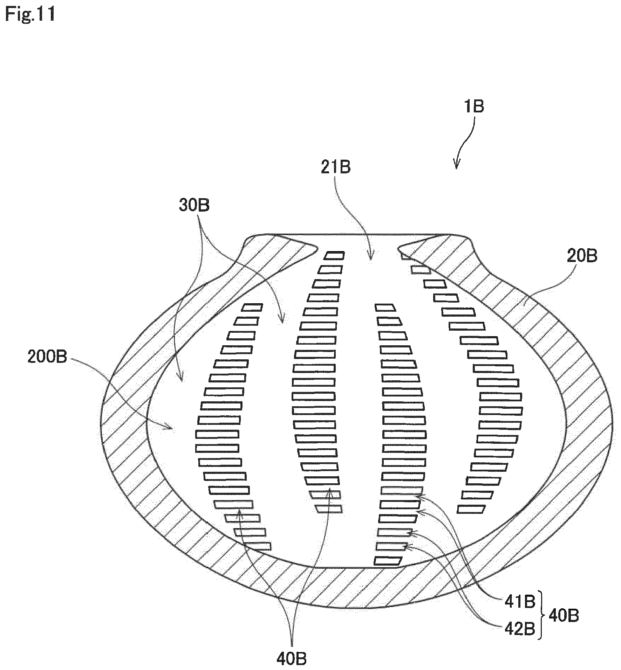

Fig. 11 is a longitudinal sectional view of an organ container 1B according to another variation. The organ container 1B has a body 20B having a shape similar to the shape of the body 20 of the organ container 1 according to the first embodiment, except for the uneven shape of an inner surface 200B.

In this organ container 1B, a plurality of first regions 30B and a plurality of second regions 40B are arranged alternatively when looking from the side of an opening 21B. Moreover, the second regions 40B adjacent to one another in the circumferential direction are arranged at positions shifted in the up-down direction when looking from the side of the opening 21B. In this way, the second regions 40B do not necessarily have to be arranged uniformly.

In the organ container 1B, the second regions 40B include a plurality of depressions 42B, and projections 41B that are arranged on a curved surface contiguous to adjacent first regions 30B. The depressions 42B are grooves extending in the horizontal direction and arranged at intervals in the up-down direction. In this way, the depressions 42B may be grooves extending in the horizontal direction.

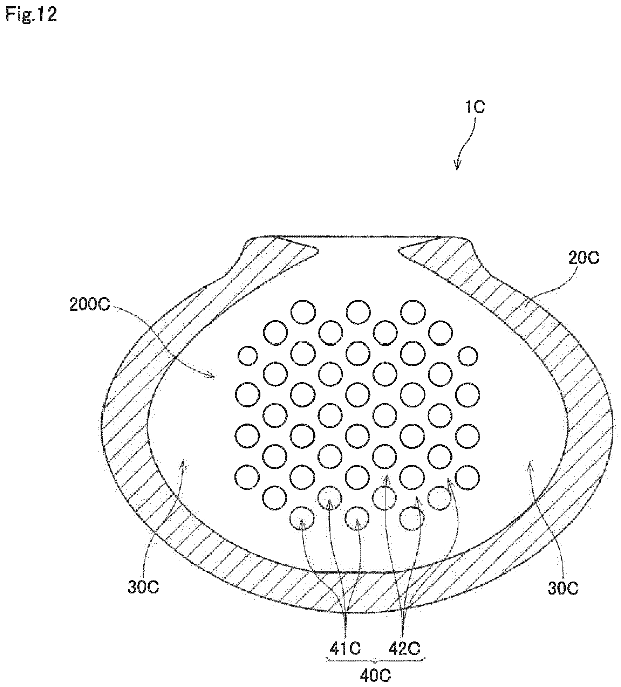

Fig. 12 is a longitudinal sectional view of an organ container 1C according to another variation. The organ container 1C has a body 20C having a shape similar to the shape of the body 20 of the organ container 1 according to the first embodiment, except for the uneven shape of an inner surface 200C.

In the organ container 1C, second regions 40C include depressions 42C that are arranged on a curved surface contiguous to adjacent first regions 30C, and a plurality of projections 41C that project inward of the depressions 42C toward the internal space of the body 20C. In this way, the depressions 42C do not necessary have to be depressed outward of adjacent first regions 30C toward the outside of the body 20C.

The projections 41C in the second regions 40C have generally circular shapes in plan view. In this way, the shapes of the projections and the depressions, which constitute the second regions, are not limited to the shape of grooves or ribs extending in any direction. The shapes of the projections and the depressions constituting the second regions may be other shapes such as circular shapes, ellipsoidal shapes, triangular shapes, square shapes including a trapezoid and a rhombus, or polygonal shapes including a pentagon or a hexagon.

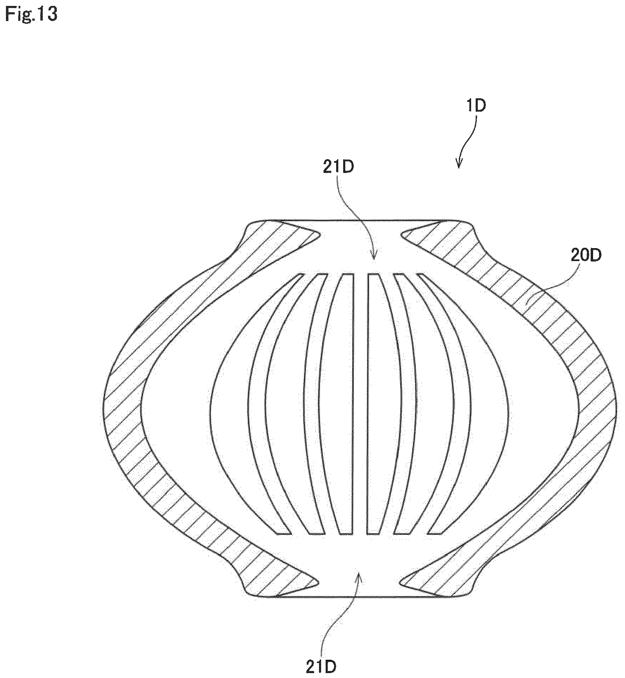

Fig. 13 is a sectional view of an organ container 1D according to another variation. This organ container 1D includes a body 20D having two openings 21D. In the embodiment and variations described above, the organ containers have a single opening. Thus, the organ containers are suitable for use in organs such as a kidney, a heart, or a lung in which blood vessels to be anastomosed during transplant operations are concentrated on one side of the organs.

In contrast, the organ container 1D illustrated in the example in Fig. 13 includes the body 20D having the two openings 21D. Thus, the organ container 1D is suitable for use in organs such as a liver, a pancreas, or a spleen in which blood vessels to be anastomosed during transplant operations extend in multiple directions. In this way, the number of openings in the body is not limited to one. In the case where the body has a plurality of openings, the sizes of the openings do not necessarily have to be the same. Openings for passing blood vessels may be smaller than an opening used to accommodate an organ.

In the case of using the organ container 1D having a plurality of openings 21D as described above, it is preferable that the organ container 1D is used in such an orientation that the openings 21D do not face downward in the vertical direction. Thus, although the two openings 21D in the example illustrated in Fig. 13 are open in the up-down direction, the present invention is not limited to this example. The two openings 21D may be provided in the upper portion and a side portion of the body 20D, or both of the two openings 21D may be provided in side portions. The positions of the openings in the body may be appropriately set in consideration of the shape of an organ to be accommodated in the container and ease of transplantation.

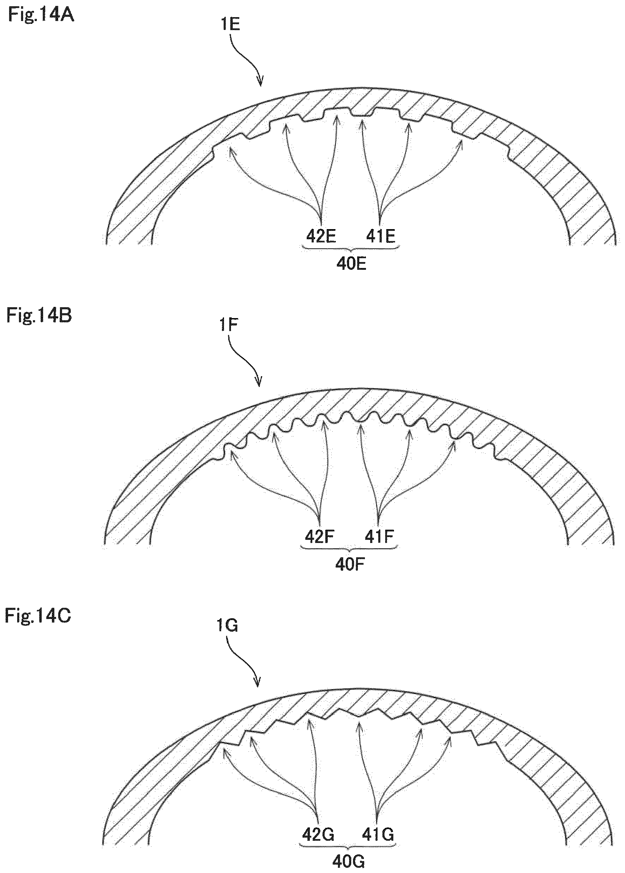

Figs. 14A, 14B, and 14C are partial cross-sectional views of organ containers IE, IF, and 1G according to other variations. In the second regions 40 according to the embodiment described above, the boundaries between adjacent projections 41 and the depressions 42 have rectangular contours in a cross section. However, the present invention is not limited to this example.

In the organ container 1E illustrated in Fig. 14A, second regions 40E include a plurality of projections 41E and a plurality of depressions 42E that are arranged alternately in a section. In this organ container IE, the boundaries between the projections 41E and the depressions 42E have smooth contours. In this way, the smooth contours of the boundaries between the projections 41E and the depressions 42E can more reliably suppress generation of remaining traces on the organ.

In the organ container 1F illustrated in Fig. 14B, second regions 40F include a plurality of projections 41F and a plurality off depressions 42F that are arranged alternatively in a section. The projections 41F have convex contours, and the depressions 42F have concave contours. Accordingly, the second regions 40F have a contour similar to a sinusoidal wave in a section. In this way, the smooth contours of the boundaries between the projections 41F and the depressions 42F can more reliably suppress generation of remaining traces on the organ.

In the organ container 1G illustrated in Fig. 14C, second regions 40G include a plurality of projections 41G and a plurality of depressions 42G that are arranged alternatively in a section. The depressions 42G are V-shaped grooves in a section, and the boundaries between the depressions 42G form the inverted V-shaped projections 41G in a section. Thus, the second regions 40G have a sectional shape similar to a triangular wave. In this way, the sectional shape of the second regions does not limited to a rectangular shape or a curved shape, and may take any of various shapes. That is, the shapes of the depressions and the projections, which constitute the second regions, are not limited to the shapes described above, and may be designed freely.

In the embodiment and variations described above, the inner surface of the thick portion is a curved surface that is smoothly contiguous to the inner surface of the other portion of the body. Thus, the thick portion protrudes only toward the outer surface of the body. However, the present invention is not limited to this example. The thick portion provided around the opening of the body may protrude toward both of the inner and outer surfaces of the body, or may protrude only toward the inner surface of the body.

In the embodiment described above, the body 20 of the organ container 1 is formed of a single kind of material, but the present invention is not limited thereto. For example, the body 20 may be configured of a plurality of layers formed of different materials. For example, a layer located close to the outer surface of the body may have a greater durometer hardness value than a layer forming the inner surface of the body that comes in contact with an organ. Alternatively, a coating may be applied to the outer surface of the body in order to prevent external damage.

A detailed structure of the organ container does not necessarily have to completely match the structure illustrated in the drawings of the present application. Each constituent element in the embodiment and variations described above may appropriately combined within a range that causes no contradictions.

## Claims

1. An organ container (1) for accommodating an organ, comprising:
a contractible and expandable pouch-shaped body (20) having an opening (21), wherein, under a no-load condition, said opening (21) has a maximum width smaller than a maximum width of said body (20),
said opening (21) is expandable up to a state in which the maximum width of said opening (21) is greater than the maximum width of said body, and
said body (20) has an inner surface (200) including:
a first region (30) having a smooth curved surface; and
a second region (40) having an uneven shape.

2. The organ container (1) according to claim 1, wherein
said inner surface has a generally ellipsoidal shape, and
said first region (30) is arranged in an end portion of said inner surface (200) in a major axis direction.

3. The organ container (1) according to claim 1, wherein
said inner surface (200) has a generally ellipsoidal shape in a cross section looking from a side of said opening (21), and
said first region (30) is arranged in an end portion of said inner surface (200) in a major axis direction and an end portion of said inner surface (200) in a minor axis direction.

4. The organ container (1) according to claim 1, wherein
said inner surface (200) has a plurality of first regions (30), each being said first region (3), and a plurality of second regions (40), each being said second region (40), in a cross section looking from a side of said opening (21), and
in the cross section looking from the side of said opening (21), said plurality of first regions (30) and said plurality of second regions (40) are arranged alternately.

5. The organ container (1) according to any one of claims 1 to 4, wherein
said second region (40) includes:
a reference plane (400) on a curved surface contiguous to said first region (30) that is adjacent to said second region; and
a depression (42) depressed toward an outside from said reference plane (400).

6. The organ container (1) according to any one of claims 1 to 4, wherein
said second region (40) has a plurality of depressions (42) and a plurality of projections (41) that are alternately arranged in a cross section, and
boundaries between said plurality of depressions (42) and said plurality of projections (41) have smooth contours.
